# FASCICULE DE BREVET EUROPEEN

(11) **EP 2 344 663 B1**
(45) Date de publication et mention de la délivrance du brevet: **23.12.2015**
(21) Numéro de dépôt: 09755980.1
(22) Date de dépôt: 07.10.2009
(51) Int. Cl.: C12Q 1/14

(54) **MILIEU REACTIONNEL POUR LES BACTERIES STAPHYLOCOCCUS AUREUS**
REAKTIONSMEDIUM FÜR STAPHYLOCOCCUS AUREUS-BAKTERIEN
REACTION MEDIUM FOR STAPHYLOCOCCUS AUREUS BACTERIA

(30) Priorité: 08.10.2008 FR 0856815
(43) Date de publication de la demande: 20.07.2011
(73) Titulaire: Biomérieux, 69280 Marcy L'etoile (FR)
(72) Inventeur: ORENGA, Sylvain, F-01160 Neuville sur Ain (FR); ROBICHON, Denis, F-01150 Blyes (FR)
(86) Numéro de dépôt international: PCT/FR2009/051909
(87) Numéro de publication internationale: WO 2010/040952

(56) Documents cités:
- WO-A-02/079486
- WO-A-2007/099254
- WO-A1-2004/063391
- FR-A- 2 790 765
- US-A1- 2003 235 879
- PERRY JOHN D ET AL: "Development and evaluation of a chromogenic agar medium for methicillin-resistant Staphylococcus aureus" JOURNAL OF CLINICAL MICROBIOLOGY, AMERICAN SOCIETY FOR MICROBIOLOGY, WASHINGTON, DC, US LNKD- DOI:10.1128/JCM.42.10.4519-4523.2004, vol. 42, no. 10, 1 octobre 2004 (2004-10-01), pages 4519-4523, XP002439826 ISSN: 0095-1137
- ATHANASOPOULOS ET AL: "Comparaison de trois milieux selectifs chromogenes pour la detection de Staphylococcus aureus resistant a la methicilline" PATHOLOGIE ET BIOLOGIE, L'EXPANSION SCIENTIFIQUE FRANCAISE, PARIS, FR LNKD- DOI:10.1016/J.PATBIO.2007.07.006, vol. 55, no. 8-9, 26 octobre 2007 (2007-10-26), pages 366-369, XP022316805 ISSN: 0369-8114

## Description

La présente invention concerne un milieu de culture pour la détection des bactéries. L'invention concerne également l'utilisation de ce milieu, ainsi qu'une méthode pour identifier des bactéries MRSA.

En 2008, le genre *Staphylococcus* regroupe quarante et une espèces et vingt-quatre sous-espèces, dont au moins dix-sept ont été retrouvées chez l'homme. La plupart de ces espèces sont des pathogènes opportunistes chez l'homme présentant un risque élevé en cas de blessure cutanée par un traumatisme ou par implantation directe d'un produit médical. D'ailleurs, l'espèce *S. aureus* est une bactérie qui se retrouve souvent chez des patients qui doivent recevoir des soins hospitaliers faisant intervenir des appareils tels que seringues, cathéters. Il y a donc un grand intérêt de détecter la présence de cette bactérie pathogène, de plus en plus impliquée dans les maladies nosocomiales.

Parmi les staphylocoques, *S. aureus* est incontestablement l'espèce la plus virulente, puisqu'elle produit un nombre important de toxines et d'enzymes extracellulaires. Elle peut être à l'origine de pathologies nombreuses et variées, allant du simple panaris, jusqu'aux infections les plus sévères comme les septicémies, endocardites, pneumopathies, infections ostéoarticulaires, dont le pronostic peut être très réservé.

En outre, seules cinq espèces : *S. aureus, Staphylococcus epidermidis, Staphylococcus haemolyticus, Staphylococcus saprophyticus* et *Staphylococcus hominis* représentent au moins 1% des souches pathogènes isolées dans des centres cliniques et, à elles cinq, plus de 98% des souches de staphylocoques les plus fréquemment rencontrées. Les autres espèces sont rarement rencontrées et peu pathogènes.
En bactériologie, il est classique d'opposer l'espèce *S. aureus,* caractérisée par la production d'une coagulase, aux autres espèces dites à coagulase négative.
Les méthodes traditionnelles pour différencier *S. aureus* de *Staphylococcus* non *aureus* reposent sur la recherche de la coagulase libre, de la DNase et sur l'obtention d'une agglutination sur latex visant à mettre en évidence la présence du facteur d'affinité pour le fibrinogène, de la protéine A et d'antigènes capsulaires.

Il faut savoir que d'autres espèces de staphylocoques, potentiellement pathogènes, sont capables d'exprimer une coagulase.

Les espèces à coagulase positive sont les suivantes : *S. aureus, Staphylococcus intermedius, Staphylococcus hyicus, Staphylococcus delphinis, Staphylococcus lutrae* et *Staphylococcus schleiferi.* Il existe des techniques de culture sur milieux sélectifs pour permettre d'évaluer la présence de *S*. *aureus.* Il s'agit des milieux suivants :
- Le milieu hypersalé de Chapman (à 75 % de NaCl) est généralement sélectif pour *S. aureus* et les staphylocoques qui hydrolysent le Mannitol. Ces bactéries font virer le milieu de rouge au jaune. Certains micro-organismes et les entérocoques du groupe D, en particulier, peuvent produire la même réaction sur le milieu ; il est donc nécessaire alors de vérifier la catalase (négative pour les streptocoques).
- Le milieu de Baird-Parker à l'oeuf (contenant du Tellurite de potassium et du Chlorure de Lithium comme agents sélectifs) est utilisé pour isoler et dénombrer les staphylocoques à coagulase positive dans les produits alimentaires et permet de mettre en évidence l'activité lécithinase. Sur ce milieu les colonies de *S. aureus,* et d'autres espèces à coagulase positive apparaissent avec un centre noir entouré d'un halo opaque. D'autres micro-organismes peuvent se développer sur ce milieu. Il s'agit principalement des groupes suivants :
   ▪ Cocci Gram + : des genres *Enterococcus* et *Listeria,*
   ▪ Bacille Gram - : des genres *Proteus* et *Pseudomonas.*
- Le milieu de Baird-Parker + RPF (plasma de lapin + fibrinogène bovin) est utilisé pour isoler et dénombrer les staphylocoques à coagulase positive dans les produits alimentaires, milieu de référence conformément à la norme ISO 6888-1 et 6888-2, et permet de mettre en évidence l'activité coagulase. Sur ce milieu les colonies de *S. aureus,* et d'autres espèces à coagulase positive apparaissent avec un centre noir entouré d'un halo opaque.

En fait, la révélation des *S. aureus*, qui utilise la technique du milieu hyper salé de Chapman, manque de sensibilité (pouvoir de mettre en évidence l'espèce recherchée lorsque celle-ci est présente en faible quantité dans un échantillon biologique à tester) et surtout de spécificité (pouvoir de détecter l'espèce recherchée dans l'échantillon biologique à tester contenant d'autres espèces).

De même, la révélation des *S. aureus,* qui utilise la technique du milieu Baird-Parker à l'oeuf, manque de sensibilité et de spécificité. Ainsi, certaines souches n'appartenant pas à l'espèce *S*. *aureus,* en particulier *Staphylococcus schleiferi* et *Staphylococcus saprophyticus,* peuvent également donner des colonies entourées d'une auréole d'éclaircissement. Il peut arriver aussi que certaines souches de *S. aureus* n'expriment pas l'activité enzymatique recherchée ou ne se développent pas sur le milieu, car ils peuvent être présents en trop faible quantité dans les échantillons biologiques et/ou inhibées par les composants trop sélectifs du milieu.

Dans le cas du milieu Baird-Parker + RPF, on observe généralement un certain nombre d'inconvénients. En premier lieu, il existe des difficultés de lecture et de sensibilité : de faux négatifs peuvent potentiellement apparaître du fait que certaines souches de *S. aureus* ne se développent pas sur ce milieu ou présentent une coagulase très faible et tardive faisant croire à la présence de staphylocoques à coagulase négative. D'autre part, des résultats faussement négatifs peuvent être également observés en raison d'interférences matricielles : en effet pour le dénombrement de faibles niveaux de contamination de *S*. *aureus,* une dilution minimale de l'échantillon est effectuée (1/10) ce qui engendre une difficulté de lecture du halo due à la présence de composés matriciels (e.g. échantillons de lait et/ou produits laitiers : couleur blanche de la caséine masquant le halo révélant la coagulase). Le milieu Baird-Parker + RPF nécessite la combinaison d'une source de thrombine et d'une source de plasminogène. Ceux-ci sont obtenus à partir du sang d'animaux, ce qui pose des problèmes de fiabilité d'approvisionnement (qualité, quantité, ...). De plus la lecture d'un halo n'est pas possible en bouillon (milieu liquide) et le contraste entre le halo et le milieu peut être réduit. Enfin en cas de colonies confluentes, il est difficile de déterminer celles qui produisent le halo de celles qui ne le produisent pas.

En second lieu, il peut également exister des problèmes de spécificité : en présence d'une flore annexe abondante (comme par exemple *Bacillus spp*), des résultats faussement positifs peuvent apparaître avec ce type de milieu. C'est par exemple le cas pour le dénombrement de *S. aureus* dans certains produits carnés (saucissons secs) et laitiers (fromages type Munster) dans lesquels *Staphylococcus xylosus* est utilisé comme ferment. Ainsi, des colonies noires entourées d'un halo apparaissent sur le milieu Baird-Parker + RPF, colonies normalement caractéristiques de *S. aureus* sur ce milieu, mais qui sont en réalité consécutives à une croissance contiguë de souches de *S. xylosus* (produisant des colonies noires sans halo) et de certaines souches de *Bacillus* possédant une coagulase (générant un halo) sur la gélose.

On comprend donc que cette révélation avec les milieux Baird-Parker, Baird-Parker + RPF et Chapman, ne donnent qu'un diagnostic présomptif et nécessitent d'autres tests de confirmation. Or, les manipulations supplémentaires, nécessaires à l'identification de *S. aureus,* augmentent la durée et le coût des analyses. Elles nécessitent une multitude de réactifs et l'intervention d'un personnel qualifié.

Il est également possible d'utiliser des milieux à base de substrats enzymatiques.

Dans un article : « Evaluation of CHROMagar Staph. aureus, a new chromogenic medium, for isolation and presumptive identification of *S. aureus* from human clinical specimens. » de Gaillot O. et al. 2000. J. Clin. Microbiol. 38, 4, 1587-1591, on décrit et évalue un milieu de culture chromogène CHROMagar (marque déposée) Staph. aureus permettant l'isolement des staphylocoques et l'identification de *S*. *aureus* à l'aide de substrats chromogènes qui procurent une coloration mauve de cette dernière espèce. Les autres espèces du même genre sont alors détectées par la coloration bleue ou incolore, en théorie. On utilise essentiellement les activités β-glucosidase, β-glucuronidase, β-galactosidase et phosphatase, ainsi qu'un inhibiteur, la Déféroxamine, qui permet la différenciation de *S. aureus* et *S. epidermidis.* Toutefois, la différentiation *S*. *aureus* des *S*. *epidermidis* est difficile, les deux espèces produisant des colonies de la même couleur sur le milieu CHROMagar (marque déposée). Ceci est dû au manque de spécificité des substrats de phosphatase qui sont positifs pour les *S. aureus* et les *S. epidermidis* et au fait que l'inhibition de ces derniers par la Déféroxamine n'est que partielle.
On peut citer également la demande WO02079486 qui décrit l'utilisation d'un substrat d'α-glucoside pour l'identification des différentes espèces de staphylocoques, qui peut être en combinaison avec un substrat d'une autre activité enzymatique. Toutefois, dans certaines conditions comme par exemple avec des souches à activité α-glucosidase réduite et en faible quantité (2 à 50 unités formant colonies), la détection de l'activité peut être tardive et nécessiter plus de 24 heures d'incubation.

S'agissant plus particulièrement des milieux sélectifs chromogènes utilisés pour la détection et/ou l'identification de *Staphylococcus aureus* résistants à la méticilline (MRSA), Anthanosopoulos et al., 2007 (Pathologie Biologie, 55(8-9) : 366-369) présente les résultats d'une évaluation des performances réalisée sur différents milieux proposés dans le commerce. Ces milieux proviennent des compagnies telles que Bio-Rad Laboratories (US), Becton Dickinson (US) et bioMérieux (FR).

De son côté, Perry et al., 2004 (J. Clin. Microbiol., 42(10) : 4519-4523) décrit un milieu chromogène destiné à la détection et/ou à l'identification de bactéries MRSA, comprenant un substrat chromogène d'alpha-glucosidase, et propose de l'améliorer en y ajoutant un antibiotique tel que la Cefoxitine, la Ciprofloxacine, l'Oxacilline et la méticilline. Une meilleure sélectivité pour les bactéries MRSA a été obtenue avec les milieux renfermant de la Cefoxitine.

L'invention se propose de résoudre les lacunes de l'état de la technique en présentant un nouveau milieu de culture sensible, spécifique, et rapide pour isoler et identifier des bactéries MRSA.
Avant d'aller plus avant, les définitions suivantes, nullement limitatives, permettront de mieux comprendre l'invention.
Au sens de la présente invention, on entend par milieu réactionnel, un milieu comprenant tous les éléments nécessaires à la survie et/ou à la croissance de microorganismes, telles que les *S. aureus.*
Ce milieu réactionnel peut soit servir uniquement de milieu de révélation, soit de milieu de culture et de révélation. Dans le premier cas, la culture des microorganismes est effectuée avant ensemencement et, dans le deuxième cas, le milieu réactionnel constitue également le milieu de culture.
Le milieu réactionnel peut être solide, semi-solide ou liquide. Par milieu solide, on entend par exemple un milieu gélifié. Préférentiellement, le milieu selon l'invention est un milieu gélifié. L'agar est l'agent gélifiant traditionnel en microbiologie pour la culture des microorganismes, mais il est possible d'utiliser de la gélatine ou de l'agarose. Un certain nombre de préparations sont disponibles dans le commerce, comme par exemple l'agar Columbia, la gélose Trypcase-soja, la gélose Mac Conkey, la gélose Sabouraud ou plus généralement celles décrites dans le Handbook of Microbiological Media (CRC Press).
Le milieu réactionnel selon l'invention peut contenir d'éventuels autres additifs comme par exemple : des peptones, un ou plusieurs facteurs de croissance, des hydrates de carbone, un ou plusieurs agents sélectifs, des solutions tampons, un ou plusieurs gélifiants... Ce milieu réactionnel peut se présenter sous forme de liquide, de gel prêt à l'emploi, c'est à dire prêt à l'ensemencement en tube, flacon, ou sur boite de Petri. Lorsque la présentation est sous forme de gel en flacon, on réalise préférentiellement une régénération (passage à 100°C) préalable du milieu avant de couler en boîte de Pétri.
Préférentiellement, le milieu selon l'invention est un milieu sélectif, c'est à dire un milieu comprenant des inhibiteurs qui privilégient la croissance des bactéries *Staphylococcus aureus.* On peut citer notamment le Chlorure de Lithium (LiCI), Azide de sodium (NaN3), Colistine, Amphotéricine, Aztréonam, Colimicine, Chlorure de Sodium (NaCl), Déféroxamine, composé vibriostatique O/129.
Ce milieu peut être également adapté à la détection des *S. aureus* résistants à la méticilline (MRSA), et comprendre un ou plusieurs antibiotiques, contre le(s)quel(s) les MRSA sont résistantes, tels que une céphalosporine ou une carbapénèmes, préférentiellement choisi parmi :
- une céphalosporine de première génération, telle que : Cefalexine, Cefaloridine, Cefalotine, Cefazoline, Cefadroxil, Cefazedone, Cefatrizine, Cefapirine, Cefradine, Cefacetrile, Cefrodaxine, Ceftezole
- une Céphalosporine de deuxième génération, telle que : Cefoxitine, Cefuroxime, Cefamandole, Cefaclor, Cefotetan, Cefonicide, Cefotiam, Loracarbef, Cefmetazole, Cefprozil, Ceforanide
- une céphalosporine de troisième génération, telle que : Cefotaxime, Ceftazidime, Cefsulodine, Ceftriaxone, Cefmenoxime, Latamoxef, Ceftizoxime, Cefixime, Cefodizime, Cefetamet, Cefpiramide, Cefoperazone, Cefpodoxime, Ceftibuten, Cefdinir, Cefditoren, Ceftriaxone, Cefoperazone
- une céphalosporine de quatrième génération, telle que Cefepime, Cefpirome
- Meropeneme, Ertapeneme, Imipeneme.

Au sens de la présente invention, le substrat d'une activité enzymatique (ou substrat enzymatique) est choisi parmi tout substrat pouvant être hydrolysé en un produit qui permet la détection, directe ou indirecte d'une activité enzymatique alpha glucosidase.

Il peut s'agir d'un substrat naturel ou synthétique. Le métabolisme du substrat provoque une variation des propriétés physico-chimiques du milieu réactionnel ou des cellules d'organismes. Cette variation peut être détectée par des méthodes physico-chimiques, notamment des méthodes optiques par l'oeil de l'opérateur ou à l'aide d'instruments, spectrométriques, électriques, magnétiques, ... Préférentiellement, il s'agit d'une variation des propriétés optiques, telles qu'une modification d'absorption, de fluorescence ou de luminescence.

Comme substrat chromogène, on peut citer notamment les substrats à base d'indoxyl, flavone, alizarine, naphtolbenzeine, nitrophénol, naphtol, catéchol, hydroxyquinoline, coumarine. Préférentiellement, le(s) substrat utilisé(s) dans la présente invention est à base d'indoxyl Comme substrat fluorescent, on peut citer notamment les substrats à base d'umbelliférone ou de coumarine, à base de résorufine, phénoxazine, naphtol, naphtylamine, 2'-hydroxyphényl-hétérocycle ou encore à base de fluorescéïne.

Comme substrat d'activité enzymatique alpha glucosidase, on peut citer plus particulièrement les substrats 5-Bromo-6-chloro-3-indoxyl-alpha-glucoside; Dihydroxyflavone-alpha-glucoside; 3,4-Cyclohexénoesculétine-alpha-glucoside; 8-Hydroxiquinoline-alpha-glucoside; 5-Bromo-4-chloro-3-indoxyl-alpha-glucoside; 5-Bromo-4-chloro-3-indoxyl-N-méthyl-alpha-glucoside; 6-Chloro-3-indoxyl-alpha-glucoside; 5-Bromo-3-indoxyl-alpha-glucoside; 5-Iodo-3-indoxyl-alpha-glucoside; 6-Fluoro-3-indoxyl-alpha-glucoside; Alizarine-alpha-glucoside; Nitrophényl-alpha-glucoside; 4-Méthylumbelliferyl-alpha-glucoside; Naphtholbenzein-alpha-glucoside; Indoxyl-N-méthyl-alpha-glucoside; Naphtyl-alpha-glucoside; Aminophényl-alpha-glucoside; Dichloroaminophényl-alpha-glucoside, Résorufine-alpha-glucoside. Préférentiellement, le substrat utilisé dans la présente invention est le 5-Bromo-4-chloro-3-indoxyl-alpha-glucoside en combinaison avec le 5-Bromo-4-chloro-3-indoxyl-N-méthyl-alpha-glucoside.

Les substrats de l'invention sont utilisables dans une large gamme de pH, notamment entre pH 5,5 et 10 préférentiellement entre pH 6,5 et 10.

La concentration en substrat est préférentiellement comprise entre 0,01 et 2 g/l, encore plus préférentiellement entre 0,02 et 0,2 g/l, et, avantageusement, elle est de 0,1 g/l. En effet, à cette concentration de substrat, on obtient un meilleur contraste de coloration.

Par échantillon biologique, on entend un échantillon clinique, issu d'un prélèvement d'aspiration bronchique, trachéale ou pulmonaire, de liquide pleural, d'un lavage broncho-alvéolaire, d'expectorations, du sang ou d'une biopsie pulmonaire, de liquide articulaire ou péricardique ; liquide biologique ou un échantillon alimentaire, issu de tout type d'aliment. Cet échantillon peut être ainsi liquide ou solide et on peut citer d'une manière non limitative, un échantillon clinique de sang, de plasma, d'urines, de fécès, de prélèvements de nez, de périnée, de gorges, de peaux, de plaies, de liquide céphalo-rachidien, un échantillon alimentaire.

A ce titre, l'invention concerne un milieu réactionnel pour la détection et/ou l'identification de bactéries MRSA comprenant une combinaison de deux substrats enzymatiques d'alpha glucosidase et un antibiotique, contre lequel les MRSA sont résistants, préférentiellement une céphalosporine, telle que la Cefoxitine. Cet antibiotique peut être en combinaison avec un autre antibiotique. Une telle combinaison est préférentiellement choisie parmi Cefoxitine / Cefotaxime ou Cefoxitine / Ertapenem. Un tel milieu est particulièrement adapté pour la détection des MRSA.

Selon un mode préféré de réalisation de l'invention, l'un des deux substrats est un substrat indoxyl-alpha-glucoside, préférentiellement le 5-Bromo-4-chloro-3-indoxyl-N-methyl-alpha-glucoside (X-N-méthyl-alpha-glucoside) ou le 5-Bromo-4-chloro-3-indoxyl- alpha-glucoside (X-alpha-glucoside).

Préférentiellement, ledit substrat indoxyl-alpha- glucoside est présent dans le milieu à une concentration comprise entre 0,01 et 2 g/l, préférentiellement entre 0,05 et 0,3 g/l. Préférentiellement, ladite combinaison de deux substrats enzymatiques d'alpha glucosidase comprend le 5-Bromo-4-chloro-3-indoxyl-N-methyl-alpha-glucoside et le 5-Bromo-4-chloro-3-indoxyl- alpha-glucoside.

Selon un mode préféré de réalisation de l'invention, ledit milieu comprend, en outre, un mélange d'inhibiteurs qui privilégie la croissance des bactéries *Staphylococcus aureus,* préférentiellement le chlorure de lithium (LiCl), l'azide de sodium (NaN3), la colistine, le composé vibriostatique O/129, l'Aztréonam, l'Amphotéricine, la colimicine, le chlorure de sodium (NaCl), Déféroxamine.

L'invention concerne également l'utilisation *in vitro* d'un milieu réactionnel tel que défini ci avant pour isoler et identifier des bactéries MRSA.

L'invention concerne enfin un procédé de détection et/ou d'identification de bactéries MRSA dans un échantillon biologique selon lequel
a) on ensemence l'échantillon biologique susceptible de contenir des bactéries MRSA sur un milieu réactionnel tel que défini ci avant
b) on incube
c) on identifie les colonies de MRSA.

L'incubation est préférentiellement réalisée à une température comprise entre 30°C et 42°C.

Les MRSA sont détectées par une activité α-glucosidase spécifique qui permet d'obtenir des colonies colorées ou fluorescentes. Les autres espèces apparaissent incolores ou d'une couleur ou fluorescence différente de celle des colonies de MRSA.

Les exemples suivants sont donnés à titre illustratif et n'ont aucun caractère limitatif. Ils permettront de mieux comprendre l'invention.

### 1. Préparation du milieu selon l'invention

Les milieux testés dans les expériences ci après étaient des milieux comprenant comme milieu de base le milieu chromID MRSA (bioMérieux réf. 43 451), et comprenant les éléments suivants
**Milieu T** : milieu témoin chromID MRSA (réf. 43 451), comprenant notamment un substrat X-N-méthyl-glucoside à une concentration de 0,1 g/l et de la Cefoxitine à 4 mg/l.
**Milieu S :** milieu T, comprenant en outre, un substrat X-alpha-Glucoside, à une concentration de 25, 37, 45 ou 50 mg/l.
**Milieu F :** milieu S (concentration X-alpha-glucoside : 45 mg/l), la Cefoxitine étant substituée par une combinaison Cefoxitine/Cefotaxime aux concentrations ci-dessous.

| | | | | |
|---|---|---|---|---|
| **[Cefoxitine] en mg/l** | 0,5 | 0,5 | 1 | 1 |
| **[Cefotaxime] en mg/l** | 0,5 | 1 | 0,5 | 1 |

**Milieu G :** milieu S (concentration X-alpha-glucoside : 45 mg/l), la Cefoxitine étant substituée par une combinaison Cefoxitine/ Ceftriaxone aux concentrations ci-dessous

| | | | | |
|---|---|---|---|---|
| **[Cefoxitine] en mg/l** | 0,5 | 0,5 | 1 | 1 |
| **[Ceftriaxone] en mg/l** | 0,5 | 1 | 0,5 | 1 |

**Milieu H :** milieu S (concentration X-alpha-glucoside : 45 mg/l), la Cefoxitine étant substituée par une combinaison Cefoxitine/ Ertapenem aux concentrations ci-dessous

| | | | | | | | | |
|---|---|---|---|---|---|---|---|---|
| **[Cefoxitine] en mg/l** | 0,5 | 0,5 | 0,5 | 0,5 | 0,75 | 0,75 | 0,75 | 0,75 |
| **[Ertapenem] en mg/l** | 0,25 | 0,5 | 0,75 | 1 | 0,25 | 0,5 | 0,75 | 1 |

**Milieu I** : milieu S (concentration X-alpha-glucoside : 45 mg/l), la Cefoxitine étant substituée par une combinaison Cefoxitine/ Cefpodoxime aux concentrations ci-dessous

| | | | | | | | | |
|---|---|---|---|---|---|---|---|---|
| **[Cefoxitine] en mg/l** | 0,5 | 0,5 | 0,5 | 0,5 | 0,75 | 0,75 | 0,75 | 0,75 |
| **[Cefpodoxime] en mg/l** | 0,5 | 1 | 1,5 | 2 | 0,5 | 1 | 1,5 | 2 |

**Milieu J :** milieu S (concentration X-alpha-glucoside : 45 mg/l), la Cefoxitine étant substituée par une combinaison Cefoxitine/ Cefoperazone aux concentrations ci-dessous

| | | | | | | | | |
|---|---|---|---|---|---|---|---|---|
| **[Cefoxitine] en mg/l** | 0,5 | 0,5 | 0,5 | 0,5 | 0,75 | 0,75 | 0,75 | 0,75 |
| **[Cefoperazone] en mg/l** | 0,5 | 0,75 | 1 | 1,5 | 0,5 | 0,75 | 1 | 1,5 |

**Milieu K :** milieu S (concentration X-alpha-glucoside : 45 mg/l), la Cefoxitine étant substituée par une combinaison Cefoxitine/Cefotaxime, aux concentrations c-dessous, comprenant en outre un mélange d'inhibiteurs des microorganismes n'appartenant pas au genre *Staphylococcus,* privilégiant la croissance des *S. aureus.*

| | | | | | | |
|---|---|---|---|---|---|---|
| **[Cefoxitine] en mg/l** | 0,75 | 0,75 | 0,75 | 0,75 | 0,75 | 0,75 |
| **[Cefotaxime] en mg/l** | 0,5 | 0,55 | 0,6 | 0,65 | 0,7 | 0,75 |

### 2. Ensemencement et lecture des milieux

Différentes souches de bactéries, toutes issues de la collection de la Demanderesse, mises en suspension dans de l'eau physiologique, ont été ensemencées pour donner des colonies isolées sur le milieu. Les boîtes ont été incubées à 37 °C pendant 48 heures. Les colonies formées ont été examinées visuellement après 18h ou 24 heures d'incubation. L'intensité de coloration était également observée selon une échelle de 0 à 4 (0 : absence de coloration, 4 : coloration très intense).

Les souches détectées correspondent aux souches formant des colonies colorées sur le milieu.

### 3. Résultats :

### 3.1 Milieu pour détecter des MRSA comprenant 2 substrats d'alpha-glucosidase

Les résultats obtenus lors de l'utilisation de un ou deux substrats d'alpha-glucosidase sont présentés dans le tableau 1.

**Tableau 1 - Intensité de coloration des colonies lors de l'utilisation de 2 substrats d'alpha glucosidase**

| | | **Milieu T** | **Milieu S [X** α **Glu] = 25 mg/l** | **Milieu [X α Glu] = 37 mg/l** | **Milieu [X** α **Glu]** = **50 mg/l** |
|---|---|---|---|---|---|
| Souches | Incubation | Intensité de coloration verte | Intensité de coloration verte | Intensité de coloration verte | Intensité de coloration verte |
| MRSA | 18h | 2 | 2 | 2,5 | 3 |
| | 24h | 2 | 2 | 3 | 3 |
| | > 40h | 2 | 2,5 | 3 | 3 |
| MRSA | 18h | 0 | 0 | 0 | 0 |
| | 24h | 2 | 2 | 2,5 | 3 |
| | > 40h | 3 | 3 | 4 | 4 |
| MRSA | 18h | 1,5 | 1,5 | 2 | 3 |
| | 24h | 2 | 2 | 4 | 4 |
| | > 40h | 2 | 3 | 4 | 4 |
| MRSA | 18h | 2 | 2 | 3 | 3 |
| | 24h | 2 | 2 | 4 | 4 |
| | > 40h | 2,5 | 2,5 | 4 | 4 |
| MRSA | 18h | 0,5 | 0,5 | 0 | 0 |
| | 24h | 1 | 0,5 | 1,5 | 2,5 |
| | > 40h | 2,5 | 3 | 4 | 4 |
| MRSA | 18h | 2 | 2 | 2,5 | 3 |
| | 24h | 2,5 | 2,5 | 2,5 | 2,5 |
| | > 40h | 2,5 | 3 | 4 | 4 |
| MRSA | 18h | 0 | 0 | 0 | 0 |
| | 24h | 1 | 1 | 3 | 4 |
| | > 40h | 2 | 3 | 4 | 4 |
| MRSA | 18h | 0 | 0 | 0 | 0 |
| | 24h | 0 | 0 | 0 | 0 |
| | > 40h | 3 | 3 | 4 | 4 |
| MRSA | 18h | 1,5 | 1,5 | 3 | 2,5 |
| | 24h | 2 | 2 | 4 | 4 |
| | > 40h | 2,5 | 3 | 4 | 4 |
| MRSA | 18h | 0 | 0 | 0 | 0 |
| | 24h | 0 | 0 | 2 | 3 |
| | > 40h | 2,5 | 3 | 4 | 4 |

L'ajout d'un deuxième substrat d'alpha-glucosidase permet d'intensifier fortement la coloration des colonies, permettant ainsi de mieux repérer les colonies de MRSA.

### 3.2 - Milieu pour détecter des MRSA comprenant deux substrats alpha-glucosidase et une combinaison d'antibiotiques choisi parmi les couples Cefoxitine/Ceftriaxone, Cefoxitine/Cefotaxime, Cefoxitine/Ertapenem, Cefoxitine/Cefoperazone ou Cefoxitine/Cefpodoxime

Les résultats obtenus lors de l'utilisation de combinaisons d'antibiotiques sont présentés dans le tableau 2.

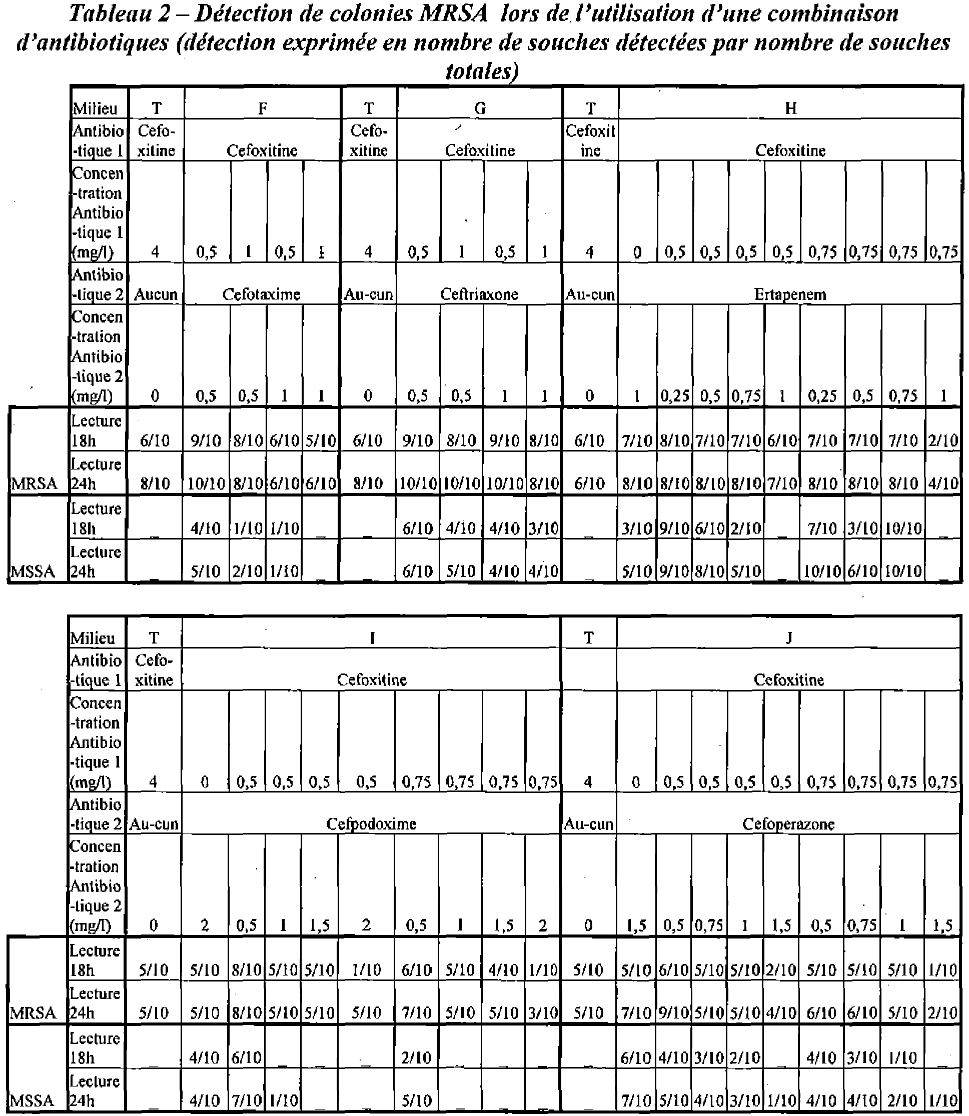

Des combinaisons d'antibiotiques ci-dessus permettent d'obtenir des performances supérieures à celles d'un milieu avec Céfoxitine seule à 4 mg/l.

### 3.3 - Milieu pour détecter des MRSA comprenant une combinaison d'antibiotiques Cefoxitine/Cefotaxime et un mélange d'inhibiteurs privilégiant la croissance des S. aureus.

Les résultats obtenus lors de l'utilisation de combinaisons d'antibiotiques sont présentés dans le tableau 3.

**Tableau 3 - Détection de colonies MRSA lors de l'utilisation d'une combinaison d'antibiotiques Cefoxitine/Cefotaxime et un mélange d'inhibiteurs (détection exprimée en nombre de souches détectées par nombre de souches totales)**

| | | | | | | | | |
|---|---|---|---|---|---|---|---|---|
| | Milieu | T | K | | | | | |
| | Antibiotique 1 | Cefoxitine | Cefoxitine | | | | | |
| | Concentration Antibiotique 1 (mg/l) | 4 | 0,5 | 0,55 | 0,6 | 0,65 | 0,7 | 0,75 |
| | Antibiotique 2 | Aucun | Cefotaxime | | | | | |
| | Concentration Antibiotique 2 (mg/l) | 0 | 0,75 | 0,75 | 0,75 | 0,75 | 0,75 | 0,75 |
| | Lecture 18h | 5/10 | 5/10 | 5/10 | 5/10 | 5/10 | 5/10 | 5/10 |
| MRSA | Lecture 24h | 9/10 | 8/10 | 9/10 | 8/10 | 8/10 | 8/10 | 9/10 |
| | Lecture 18h | _ | _ | _ | _ | _ | _ | _ |
| MSSA | Lecture 24h | _ | 3/10 | 2/10 | 1/10 | _ | _ | _ |

La combinaison d'antibiotiques Cefoxitine/Cefotaxime associée à un mélange d'inhibiteurs privilégiant la croissance des *S. aureus* permettait d'obtenir une excellente spécificité et sensibilité, en particulier lors de l'utilisation d'une concentration en Cefoxitine et en Cefotaxime de 0,75 mg/l.

## Revendications

1. Milieu réactionnel pour la détection et/ou l'identification de bactéries MRSA comprenant une combinaison de deux substrats enzymatiques d'alpha glucosidase et un antibiotique, contre lequel les MRSA sont résistants, préférentiellement une céphalosporine, telle que la Cefoxitine.

2. Milieu réactionnel selon la revendication 1 **caractérisé en ce qu'**il comprend un deuxième antibiotique, contre lequel les MRSA sont résistants.

3. Milieu réactionnel selon la revendication 1 ou 2 **caractérisé en ce qu'**un des deux substrats est un substrat indoxyl alpha glucoside, préférentiellement le 5-Bromo-4-chloro-3-indoxyl-N-methyl-alpha-glucoside ou le 5-Bromo-4-chloro-3-indoxyl- alpha-glucoside.

4. Milieu réactionnel selon la revendication 3 **caractérisé en ce que** ledit substrat indoxyl alpha glucoside est présent dans le milieu à une concentration comprise entre 0,01 et 2 g/l, préférentiellement entre 0,02 et 0,3 g/l.

5. Milieu réactionnel selon l'une quelconque des revendications 1 à 4 **caractérisé en ce que** ladite une combinaison de deux substrats enzymatiques d'alpha glucosidase comprend le 5-Bromo-4-chloro-3-indoxyl-N-methyl-alpha-glucoside et le 5-Bromo-4-chloro-3-indoxyl- alpha-glucoside.

6. Milieu réactionnel selon l'une quelconque des revendications 1 à 5 **caractérisé en ce qu'**il comprend, en outre, un mélange d'inhibiteurs qui privilégie la croissance des bactéries *Staphylococcus aureus,* préférentiellement le LiCl, composé vibriostatique O/129, Aztréonam, et. Amphotéricine.

7. Utilisation *in vitro* d'un milieu réactionnel selon l'une quelconque des revendications 1 à 6 pour isoler et identifier des bactéries MRSA.

8. Procédé de détection et/ou d'identification de bactéries MRSA dans un échantillon biologique selon lequel
a) on ensemence l'échantillon biologique susceptible de contenir des bactéries MRSA sur un milieu réactionnel tel que défini dans les revendications 1 à 6 ;
b) on incube ;
c) on identifie les colonies de MRSA.

## Patentansprüche

1. Reaktionsmedium für den Nachweis und/oder die Identifikation von MRSA-Bakterien, das eine Kombination von zwei Enzymsubstraten der alpha-Glucosidase und ein Antibiotikum, gegen das die MRSA resistent sind, vorzugsweise ein Cephalosporin wie Cefoxitin, umfasst.

2. Reaktionsmedium nach Anspruch 1, **dadurch gekennzeichnet, dass** es ein zweites Antibiotikum, gegen das die MRSA resistent sind, umfasst.

3. Reaktionsmedium nach Anspruch 1 oder 2, **dadurch gekennzeichnet, dass** eines der beiden Substrate ein Indoxyl-alpha-Glucosidsubstrat, vorzugsweise 5-Brom-4-chlor-3-indoxyl-N-methyl-alpha-glucosid oder 5-Brom-4-chlor-3-indoxyl-alpha-glucosid, ist.

4. Reaktionsmedium nach Anspruch 3, **dadurch gekennzeichnet, dass** das Indoxyl-alpha-Glucosidsubstrat in einer Konzentration zwischen 0,01 und 2 g/l, vorzugsweise zwischen 0,02 und 0,3 g/l, in dem Medium vorliegt.

5. Reaktionsmedium nach einem der Ansprüche 1 bis 4, **dadurch gekennzeichnet, dass** die eine Kombination von zwei Enzymsubstraten der alpha-Glucosidase 5-Brom-4-chlor-3-indoxyl-N-methyl-alpha-glucosid und 5-Brom-4-chlor-3-indoxyl-alpha-glucosid umfasst.

6. Reaktionsmedium nach einem der Ansprüche 1 bis 5, **dadurch gekennzeichnet, dass** es weiterhin eine Mischung von Hemmstoffen, die das Wachstum von *Staphylococcus-aureus-Bakterien* fördert, vorzugsweise LiCl, die vibriostatische Verbindung O/129, Aztreonam und Amphotericin, umfasst.

7. In-vitro-Verwendung eines Reaktionsmediums nach einem der Ansprüche 1 bis 6 zum Isolieren und Identifizieren von MRSA-Bakterien.

8. Verfahren für den Nachweis und/oder die Identifikation von MRSA-Bakterien in einer biologischen Probe, bei dem man
a) die biologische Probe, die MRSA-Bakterien enthalten kann, auf ein wie in den Ansprüchen 1 bis 6 definiertes Reaktionsmedium impft;
b) inkubiert;
c) die MRSA-Kolonien identifiziert.

## Claims

1. Reaction medium for detecting and/or identifying MRSA bacteria, comprising a combination of two enzymatic substrates of alpha-glucosidase and an antibiotic, against which the MRSAs are resistant, preferentially a cephalosporin, such as cefoxitin.

2. Reaction medium according to Claim 1, **characterized in that** it comprises a second antibiotic, against which the MRSAs are resistant.

3. Reaction medium according to Claim 1 or 2, **characterized in that** one of the two substrates is an indoxyl-alpha-glucoside substrate, preferentially 5-bromo-4-chloro-3-indoxyl-N-methyl-alpha-glucoside or 5-bromo-4-chloro-3-indoxyl-alpha-glucoside.

4. Reaction medium according to Claim 3, **characterized in that** said indoxyl-alpha-glucoside substrate is present in the medium at a concentration of between 0.01 and 2 g/l, preferentially between 0.02 and 0.3 g/l.

5. Reaction medium according to any one of Claims 1 to 4, **characterized in that** said combination of two enzymatic substrates of alpha-glucosidase comprises 5-bromo-4-chloro-3-indoxyl-N-methyl-alpha-glucoside and 5-bromo-4-chloro-3-indoxyl-alpha-glucoside.

6. Reaction medium according to any one of Claims 1 to 5, **characterized in that** it also comprises a mixture of inhibitors which favours the growth of *Staphylococcus aureus* bacteria, preferential LiCl, the vibriostatic compound O/129, aztreonam and amphotericin.

7. *In vitro* use of a reaction medium according to any one of Claims 1 to 6, for isolating and identifying MRSA bacteria.

8. Method for detecting and/or identifying MRSA bacteria in a biological sample, according to which
a) the biological sample which may contain MRSA bacteria is inoculated on a reaction medium as defined in Claims 1 to 6;
b) incubation is carried out;
c) the MRSA colonies are identified.
